# FASCICULE DE BREVET EUROPEEN

(11) **EP 1 068 555 B1**
(45) Date de publication et mention de la délivrance du brevet: **07.01.2004**
(21) Numéro de dépôt: 99911858.1
(22) Date de dépôt: 01.04.1999
(51) Int. Cl.: G02C 7/02

(54) **SYSTEME OPTIQUE, NOTAMMENT LENTILLE INTRAOCULAIRE, LENTILLE DE CONTACT**
OPTISCHES SYSTEM, INSBESONDERE INTRAOKULARE ODER KONTAKTLINSE
OPTICAL SYSTEM, IN PARTICULAR INTRAOCULAR LENS, CONTACT LENS

(30) Priorité: 02.04.1998 FR 9804109
(43) Date de publication de la demande: 17.01.2001
(73) Titulaire: Ioltech, 17000 La Rochelle (FR)
(72) Inventeur: FEURER, Bernard, F-31450 Montlaur (FR); MAUZAC, Monique, F-31000 Toulouse (FR)
(74) Mandataire: Santarelli
(86) Numéro de dépôt international: PCT/FR1999/000764
(87) Numéro de publication internationale: WO 1999/052009

(56) Documents cités:
- EP-A- 0 407 294
- US-A- 5 172 143
- US-A- 5 258 024
- YASUHIRO KOIKE ET AL: "GRADIENT-INDEX CONTACT LENS" APPLIED OPTICS, vol. 34, no. 22, 1 août 1995 (1995-08-01), pages 4669-4673, XP000518143 ISSN: 0003-6935

## Description

La présente invention concerne les systèmes optiques, notamment les systèmes optiques centrés tels que les lentilles intraoculaires, les lentilles de contact, etc.

On sait que l'oeil humain est un système optique complexe dont le rôle est de transmettre au cerveau les images qui lui parviennent. L'un des éléments essentiels est le cristallin. Situé en arrière de l'iris, le cristallin est une masse gélatineuse transparente contenue dans le sac cristallinien.

L'opacification du cristallin peut survenir avec l'âge (cataracte). On est alors contraint de retirer le cristallin déficient et de le remplacer par un cristallin artificiel ou par une lentille intraoculaire.

Les cristallins artificiels connus à ce jour sont essentiellement réalisés en matériaux acryliques, par exemple du polyméthyl-méthacrylate ou ses copolymères, ou en dérivés silicones. Ils ont des indices de réfraction relativement faibles. Pour les silicones, on a actuellement des indices de réfraction compris entre 1,41 et 1,46 dans le meilleur des cas. Pour les corrections fortes, il faut donc utiliser des lentilles intraoculaires dont les faces ont une courbure importante et qui, de ce fait, ont une grande épaisseur au niveau de leur axe optique.

Pour obtenir la meilleure correction et ne pas induire des défauts d'astigmatisme, il est en outre nécessaire d'introduire la lentille intraoculaire par une incision de la plus faible dimension possible. Pour ce faire, on recherche des matériaux souples et du plus grand indice de réfraction possible de façon à obtenir une lentille intraoculaire très fine.

L'oeil en bonne santé dispose d'un cristallin capable, sous l'action de muscles, les zonules, qui mobilisent le sac cristallinien, de modifier son rayon de courbure en sorte de s'adapter à la vision de près ou à la vision de loin.

Le remplacement du cristallin par une lentille intraoculaire ne permet plus l'accommodation.

Un des objectifs de la présente invention est de réaliser un système optique du type lentille intraoculaire qui pallie les inconvénients de ceux de l'art antérieur.

Plus précisément, la présente invention a pour objet un système optique, notamment lentille intraoculaire, lentille de contact, selon la revendication 1.

Une autre application est la réalisation de lentilles de contact à double foyer, permettant une correction simultanée de deux défauts visuels (myopie et presbytie par exemple) :
- soit par juxtaposition de deux matériaux, un central, un périphérique, de nature similaire, mais d'indices différents, grâce à des taux de greffage différents sur une même matrice;
- soit par juxtaposition de deux domaines différents d'un même matériau, les deux domaines présentant des indices de réfraction différents grâce à une orientation moléculaire;
- soit par réalisation d'un matériau dont l'indice varie sous l'effet d'une contrainte mécanique, la pression des paupières par exemple.

D'autres caractéristiques et avantages de la présente invention apparaîtront au cours de la description suivante donnée en regard des dessins annexés à titre illustratif, mais nullement limitatif, dans lesquels:

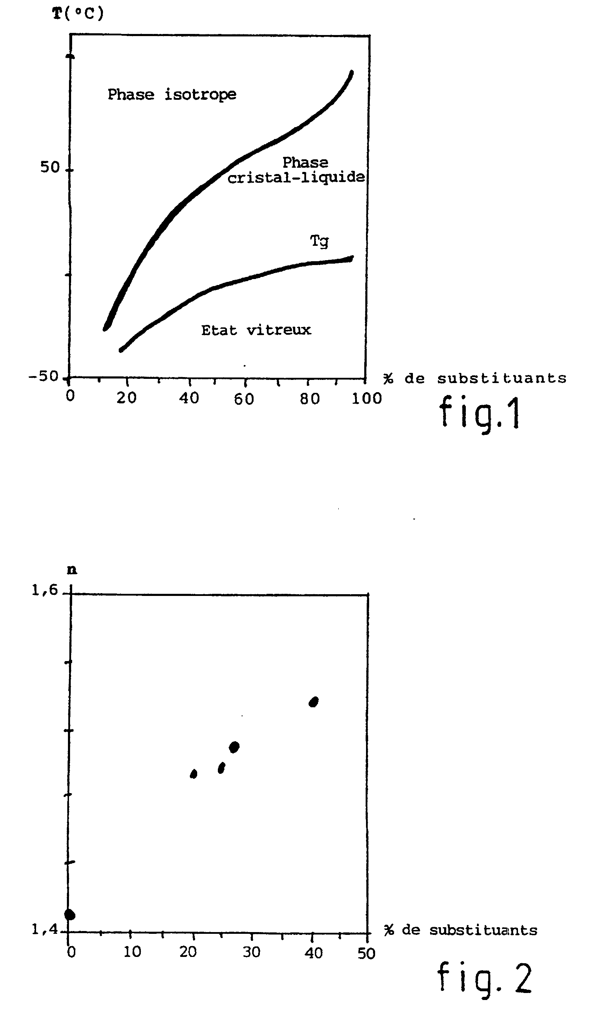

Les figures 1 et 2 représentent des graphiques permettant d'expliciter les variations de propriétés de matériaux utilisés pour la réalisation du système optique du type lentille intraoculaire selon l'invention en fonction de la composition de ces matériaux, le graphique selon la figure 1 représentant l'évolution des températures de transition vitreuse en fonction du taux de substituants, et le graphique selon la figure 2 représentant l'évolution de l'indice de réfraction n en fonction du taux de substituants.

Le système optique du type lentille intraoculaire selon l'invention est réalisé en un matériau dont l'indice de réfraction optique présente des variations dans au moins une direction donnée.

Dans un premier mode de réalisation, ce matériau est homogène et présente un indice de réfraction n élevé variable selon sa composition chimique.

En effet, pour une molécule donnée, la réfraction molaire R est, en première approximation, une fonction additive des contributions des divers éléments présents dans la molécule. Parmi les groupes chimiques courants, ceux qui ont les effets les plus importants dans l'augmentation de R sont principalement le soufre, les halogènes, en particulier le chlore, le brome et l'iode, et les noyaux aromatiques.

L'indice de réfraction n de la molécule augmente avec R de telle sorte que ce sont les molécules contenant les éléments précédemment cités qui ont les indices les plus élevés.

| | | |
|---|---|---|
| Exemples | benzène | n = 1,498 |
| | o-dichlorobenzène | n = 1,551 |
| | disulfure de carbone | n = 1,628 |
| | diiodométhane | n = 1,749 |

De même, l'addition de groupes d'indice de réfraction n élevé sur un polymère accroît l'indice de réfraction du matériau.

A titre d'exemple, on citera le cas de silicones substitués par des entités 9-vinyl anthracène. L'indice de réfraction du matériau obtenu augmente avec le taux de substituants:
- sans substituant: n = 1,403
- avec 94% de substituants: n = 1,690

Les températures de transition vitreuse T_{g} augmentent elles aussi avec le taux de substitution du fait de la rigidité des noyaux aromatiques:
- sans substituant: T_{g} = -130°C
- avec 94% de substituants: T_{g} = entre 10°C et 20°C

Le processus de fabrication du matériau homogène et présentant un indice de réfraction n élevé et variable selon sa composition chimique, nécessaire à la réalisation d'une lentille intraoculaire selon l'invention comporte les deux étapes suivantes:

En premier lieu, des groupes choisis dans ceux décrits précédemment, en particulier des noyaux aromatiques dont la présence confère en outre au matériau obtenu une capacité de filtration des rayonnements ultra violets, propriété indispensable pour une lentille intraoculaire de bonne qualité, sont fixés sur les polymères utilisés pour les lentilles et cristallins artificiels, cette fixation étant obtenue par l'intermédiaire d'une partie flexible afin de perturber la température T_{g} le moins possible.

Exemples:
Substituant de type [1] : 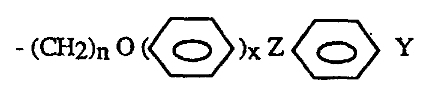
Substituant de type [2] : 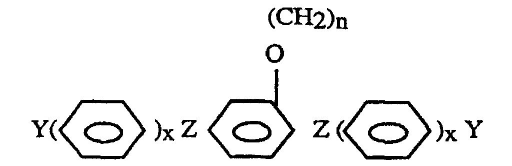 avec Z = OCO, COO, - , ...
   Y = H, OCₘH₂ₘ₊₁, CₘH₂ₘ₊₁, ...
   avec n>2, m≥1, x=1, 2, 3, ...

Ensuite, le taux de substitution est modifié de façon continue, et donc aussi l'indice de réfraction du matériau, pour obtenir des copolymères à proportion modulable de motifs substitués et de motifs non substitués. Dans le cas des silicones, il est nécessaire de réaliser au préalable le copoly(méthylhydrogéno-diméthyl)siloxane de composition variable.

Deux exemples sont donnés ci-après, l'un à partir de support silicone, l'autre à partir de support acrylate, le substituant choisi correspondant à la formule [1] ci-dessus où n=4, Z=OCO, Y=OCₘH₂ₘ₊₁ avec m=1, x=1.

Dans le cas du premier exemple, celui avec un support silicone, le substituant doit posséder une liaison vinylique terminale:
Exemple :

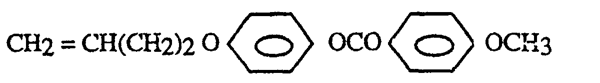

Ce groupe peut être obtenu en deux étapes : réaction du 4-bromobutène sur l'hydroquinone, puis estérification par l'acide p-méthoxybenzoïque.

La chaîne principale siloxane présente une distribution statistique de motifs substituables méthylhydrogénosiloxane et de motifs non substituables diméthylsiloxane en proportion variable. Ces copolymères sont obtenus par redistribution acido-catalysée de motifs diméthylsiloxane introduits en quantité adéquate par l'intermédiaire de l'octaméthylcyclotétrasiloxane et de motifs méthylhydrogénosiloxane amenés par des homopolyméthyl-hydrogénosiloxanes.

Le substituant est fixé sur la chaîne principale par hydrosilylation à 60°C en présence d'un solvant. Il est introduit en défaut par rapport aux motifs méthylhydrogénosiloxane (de 5% à 15%) afin de permettre une réaction ultérieure des motifs excédentaires lors de l'étape de réticulation.

En fin de réaction d'hydrosilylation, le polymère est débarrassé de la quasi totalité du solvant par évaporation sous vide à température ambiante. Il est ensuite mélangé à un agent réticulant, et le reste de solvant est évaporé sous vide.

L'agent réticulant est préférentiellement une chaîne flexible et se termine par deux extrémités vinyliques. Sa proportion est telle que la quantité de liaisons vinyliques correspond à la quantité de motifs méthylhydrogénosiloxanes laissés libres.

Exemple d'agent réticulant:

CH₂=CH(CH₂)ₚCH=CH₂

p=2 à 20

CH₂=CH(Si(CH₃)₂O)_{q}CH=CH₂

q=2 à 10

Le mélange polymère/agent réticulant est coulé dans un moule traité de façon à ce que le matériau ne colle pas aux parois. Le moule est mis à 60°C dans une étuve pendant plusieurs heures afin d'obtenir un polymère réticulé que l'on démoule.

Ce produit peut être lavé en le gonflant par un solvant, afin d'éliminer les éventuelles molécules qui n'auraient pas réagi, puis en le séchant lentement.

Dans le second exemple, celui avec un support acrylate, le monomère acrylate ou méthacrylate, porteur du substituant choisi, doit être synthétisé:

Exemple : 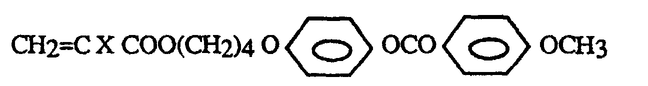 avec X = H, CH₃

Ce groupe peut être obtenu en quatre étapes: réaction du 4-bromobutanol dans lequel la fonction alcool a été protégée, sur l'hydroquinone ; estérification par l'acide p-méthoxybenzoïque ; déprotection de la fonction alcool ; estérification entre cette fonction alcool et le groupe carboxylique de l'acide acrylique ou méthacrylique.

Un monomère difonctionnel présentant une fonction acrylate ou méthacrylate aux deux extrémités doit également être synthétisé. Il peut être obtenu selon le schéma suivant: réaction du 4-bromobutanol dans lequel la fonction alcool a été protégée, sur l'acide hydroxybenzoïque ; estérification par le produit de la réaction du 4-bromobutanol dans lequel la fonction alcool a été protégée, sur l'hydroquinone ; déprotection des fonctions alcool ; estérification de ces fonctions alcool par les fonctions carboxylique de l'acide acrylique ou méthacrylique.

D'autres monomères difonctionnels peuvent être utilisés: éthylène glycol diméthacrylate ; triéthylène glycol diméthacrylate ; tétraéthylène glycol diméthacrylate ; 1,6 hexane diol diméthacrylate ; 1,12 dodécane diol diméthacrylate.

La polymérisation est déclenchée par chauffage ou irradiation UV en présence d'un initiateur (azobis isobutyronitrile par exemple) ou par tout autre système courant (accélérateur chimique, irradiation micro onde).

L'obtention de matériaux réticulés de proportion variable en substituants est possible en mélangeant, préalablement à la réaction de polymérisation, un ou plusieurs monomères non substitués (acrylate de méthyle, méthacrylate de méthyle, hydroxy éthyl méthacrylate par exemple) avec les monomères précédents monofonctionnels et bifonctionnels en proportion adéquate. L'hydroxy éthyl méthacrylate (HEMA) apporte un caractère hydrophile au matériau jusqu'à obtenir un taux d'hydratation de 40% pour un homopolymère. On peut aussi y associer des co-monomères encore plus hydrophiles tel que la N-vinyl pyrrolidone (VP) par exemple.

Les cristallins ou lentilles peuvent être obtenus soit par usinage des matériaux finaux, soit par la réalisation de la dernière étape (polymérisation/réticulation) dans un moule. Dans le cas où le monomère de base présente des qualités hydrophiles, le matériau final peut être gonflé en milieu aqueux et devenir plus ou moins pliable en fonction de sa composition.

Les matériaux ainsi obtenus présentent, par rapport aux silicones ou acrylates de base, des propriétés qui permettent la réalisation de cristallins artificiels, de lentilles intraoculaires, ou de lentilles de contact selon l'invention.

En effet, leur indice de réfraction n et leur température de transition vitreuse T_{g} sont plus élevés et varient selon leur composition chimique. Notamment, ils augmentent avec l'augmentation des proportions des substituants.

Un exemple de cette évolution est illustré sur les figures 1 et 2 pour les matériaux silicones dont le mode de synthèse a été donné ci-avant .

Dans cet exemple, l'agent réticulant est une chaîne alkyle; trois longueurs de chaînes d'agent réticulant ont été étudiées correspondant à 10, 16 ou 22 carbones; trois proportions différentes de cet agent réticulant ont été introduites (5, 10, 15%). Ces deux paramètres ont peu d'influence sur l'évolution de l'indice de réfraction ou de la température de transition vitreuse, comme on peut le voir sur les figures 1 et 2.

L'indice de réfraction augmente par contre très rapidement avec le taux de substituants, figure 2, puisque avec 40% de substituants on obtient des indices supérieurs à 1,53.

L'évolution de la température de transition vitreuse, figure 1, est plus lente. Même avec une substitution totale, T_{g} reste inférieure à la température ambiante.

Les propriétés mécaniques sont quant à elles relativement peu perturbées par les substituants. Par exemple: le module élastique sous cisaillement (G') à fréquence nulle:

| | |
|---|---|
| silicone non modifié | G' = 10⁵ Pa |
| silicone avec plus de 85% de substituants | G' = 4.10⁴ Pa |

Selon un deuxième mode de réalisation, le matériau dans lequel est réalisée la lentille intraoculaire selon l'invention est un matériau hétérogène d'indice élevé et variable dans le matériau.

Les substituants aromatiques proposés ci-dessus sont des cristaux-liquides thermotropes. Ils apportent des propriétés mésomorphes au polymère qui les porte, c'est-à-dire en particulier des propriétés d'orientation moléculaire: dans un domaine de température donné, ces substituants s'orientent très facilement sous l'effet par exemple d'un champ magnétique ou électrique. Cette orientation est ensuite "figée" par le processus de réticulation.

Sous l'effet de l'orientation comme mentionné ci-dessus, l'indice de réfraction devient anisotrope. Il est donc possible, par orientation des substituants, de modifier l'indice de réfraction dans une direction donnée.

Selon la présente invention, le système optique est obtenu à partir d'un même polymère, silicone ou acrylate ou méthacrylate par exemple, en réalisant des lots d'indices différents obtenus par des orientations des substituants dans des directions différentes.

Les orientations peuvent être obtenues en plaçant le polymère substitué (dans le cas des silicones) ou les divers monomères, substitués ou non, (dans le cas des acrylates) dans un champ magnétique faible d'environ 1 Tesla ou dans un champ électrique, ou par un traitement de surface du dispositif permettant de fabriquer le matériau ou la lentille dans sa forme finale. La réticulation (dans le cas des silicones) ou la polymérisation/réticulation (dans le cas des acrylates) sont réalisées par traitement thermique par exemple, sous ce champ orienteur.

Ces lots de même nature chimique sont parfaitement compatibles. Ils pourront être assemblés de façon à former des lentilles ou des cristallins de zones d'accommodation différentes. Par exemple, on pourra réaliser une lentille intraoculaire en deux parties: une zone optique centrale adaptée à la vision de près et une zone périphérique adaptée à la vision de loin.

Selon un troisième mode de réalisation, le matériau utilisé pour la réalisation du système optique du type lentille intraoculaire selon l'invention est un matériau homogène d'indice élevé et variable par effet mécanique, permettant en cela l'accommodation.

Selon une caractéristique de l'invention, le matériau dans lequel est réalisé le système optique est un polymère cristaux-liquides tridimensionnel dont l'orientation des entités mésomorphes peut facilement être obtenue par effet mécanique.

On pourra par exemple d'abord réaliser des polymères cristaux-liquides réticulés sans orientation préalable des mésogènes. A partir de ce matériau, seront alors réalisés, par exemple par polymérisation/réticulation dans un moule ou par usinage selon les propriétés du matériau, des cristallins artificiels ou des lentilles intraoculaires. Les zonules exercent une contrainte mécanique qui se répercute, par l'intermédiaire du sac cristallinien, sur le cristallin. Cette contrainte modifie l'orientation des substituants cristaux-liquides et donc l'indice de réfraction dans la direction de vision. De même dans le cas des lentilles de contact, une pression des paupières peut donner des déformations mécaniques nécessaires à la réorientation moléculaire et donc faire varier l'indice de réfraction et par contre coup la puissance de la lentille.

Il est aussi possible de donner à ces matériaux lors de leur réalisation, une orientation préalable des substituants, qui sera modifiée sous l'effet de compressions ou d'étirements transmis au sac par les zonules.

Pour que le matériau sans orientation préalable des mésogènes, soit transparent, ou qu'un matériau préalablement orienté demeure transparent après désorientation, il est placé en phase isotrope dans les conditions d'utilisation. De plus, pour obtenir une orientation suffisante sous contrainte et donc une modification significative de l'indice de réfraction, il est nécessaire de mettre en oeuvre le procédé dans une plage de température d'environ 10°C au-dessus de la température T_{I} à laquelle l'échantillon devient isotrope. Cette obligation impose une limite supérieure au taux de substitution comme illustré sur la Figure 1. Dans l'exemple choisi, un siloxane modifié à environ 35% conviendrait parfaitement : il est isotrope vers 35°C avec un indice de réfraction supérieur à 1,51 (Figure 1).

En phase isotrope, la variation d'indice est d'autant plus importante que la température d'utilisation est proche de T_{I}. Un exemple de la différence d'indice entre deux directions perpendiculaires, Δn, induite par une contrainte mécanique est donné ci-dessous. Le composé choisi correspond à un méthacrylate substitué par divers groupes du type [2] défini ci-avant:

| | |
|---|---|
| à T_{I} + 4°C, | Δn = 6.10⁻³ pour une contrainte de 5.10⁻² N.mm⁻² |
| | Δn = 2.10⁻³ pour une contrainte de 2.10⁻² N.mm⁻² |
| à TI + 25°C, | Δn = 1.10⁻³ pour une contrainte de 5.10⁻² N.mm⁻² |
| | Δn = 0,3.10⁻³ pour une contrainte de 2.10⁻² N.mm⁻² |

## Revendications

1. Système optique, du type lentille intraoculaire, réalisée en un matériau homogène dont l'indice de réfraction optique présente des variations suivant au moins une direction donnée, **caractérisé en ce que** ledit indice de réfraction est variable sous l'action d'un effet mécanique et **en ce que** ledit matériau homogène est constitué par au moins un matériau polymère cristaux-liquides tridimentionnel.

2. Système optique selon la revendication 1, **caractérisé en ce que** l'effet mécanique est exercé par les zonules.

3. Système optique selon l'une des revendications 1 et 2, **caractérisé en ce qu'**il consiste en une lentille intraoculaire.

4. Système optique consistant en une lentille de contact réalisée en un matériau homogène dont l'indice de réfraction optique présente des variations suivant au moins une direction donnée, **caractérisé en ce que** ledit indice de réfraction est variable sous l'action d'un effet mécanique et **en ce que** ledit matériau homogène est constitué par au moins un matériau polymère cristaux-liquides tridimentionnel.

5. Système optique selon la revendication 4, **caractérisé en ce que** l'effet mécanique est exercé par pression des paupières.

## Patentansprüche

1. Optisches System des Typs Interokularlinse, aus einem homogenen Linsenmaterial, dessen optischer Brechungsindex in mindestens einer vorgegebenen Richtung Veränderungen zeigt, **dadurch gekennzeichnet, dass** der Brechnungsindex unter mechanischer Einwirkung veränderbar ist, und dass das homogene Material aus mindestens einem polymeren Material dreidimensionaler Flüssigkristalle gebildet ist.

2. Optisches System nach Anspruch 1, **dadurch gekennzeichnet, dass** die mechanische Einwirkung durch die Zonula ausgeübt ist.

3. Optisches System nach einem der Ansprüche 1 und 2, **dadurch gekennzeichnet, dass** es aus einer Interokularlinse gebildet ist.

4. Optisches System, gebildet aus einer Kontaktlinse aus einem homogenen Linsenmaterial, dessen optischer Brechnungsindex in mindestens einer vorgegebenen Richtung Veränderungen zeigt, **dadurch gekennzeichnet, dass** der Brechnungsindex unter mechanischer Einwirkung veränderbar ist, und dass das homogene Material aus mindestens einem polymeren Material dreidimensionaler Flüssigkristalle gebildet ist.

5. Optisches System nach Anspruch 4, **dadurch gekennzeichnet, dass** die mechanische Einwirkung durch einen Druck der Augenlider ausgeübt ist.

## Claims

1. An optical system of intraocular lens type made from a homogenous lens material of which the optical refractive index varies in at least one given direction, **characterized in that** said refractive index is variable under the action of a mechanical effect and **in that** said homogenous material is constituted by at least one three-dimensional liquid crystal polymer.

2. An optical system according to claim 1, **characterized in that** the mechanical effect is applied by the zonules.

3. An optical system according to one of claims 1 and 2, **characterized in that** it consists of an intraocular lens.

4. An optical system consisting of a contact lens made from a homogenous material of which the optical refractive index varies in at least one given direction, **characterized in that** said refractive index is variable under the action of a mechanical effect and **in that** said homogenous material is constituted by at least one three-dimensional liquid-crystal polymer.

5. An optical system according to claim 4, **characterized in that** the mechanical effect is applied by pressure of the eye-lids.
